# EUROPÄISCHE PATENTSCHRIFT

(11) **EP 1 041 925 B1**
(45) Veröffentlichungstag und Bekanntmachung des Hinweises auf die Patenterteilung: **17.09.2003**
(21) Anmeldenummer: 98966668.0
(22) Anmeldetag: 23.12.1998
(51) Int. Cl.: A61B 5/022

(54) **VERFAHREN UND MESSGERÄT ZUR BESTIMMUNG DES BLUTDRUCKS**
METHOD AND MEASURING DEVICE FOR DETERMINING BLOOD PRESSURE
PROCEDE ET APPAREIL DE MESURE POUR DETERMINER LA PRESSION SANGUINE

(30) Priorität: 24.12.1997 DE 19757974
(43) Veröffentlichungstag der Anmeldung: 11.10.2000
(60) Teilanmeldung: 03015712.7
(73) Patentinhaber: Braun GmbH, 61476 Kronberg (DE)
(72) Erfinder: FREUND, Dirk, D-65779 Kelkheim (DE); SCHNAK, Fred, D-61476 Kronberg (DE); GIERSIEPEN, Martin, D-61440 Oberursel (DE); KRESSMANN, Frank, D-65824 Schwalbach (DE); HARTTMANN, Brigitte, D-65527 Niedernhausen (DE)
(86) Internationale Anmeldenummer: EP9808429
(87) Internationale Veröffentlichungsnummer: WO99033395

(56) Entgegenhaltungen:
- DE-U- 29 612 412
- SU-A- 1 477 377
- SU-A- 1 807 863
- US-A- 4 338 950
- US-A- 4 870 973
- US-A- 5 111 826
- PATENT ABSTRACTS OF JAPAN vol. 096, no. 012, 26. Dezember 1996 & JP 08 215161 A (OMRON CORP), 27. August 1996
- PATENT ABSTRACTS OF JAPAN vol. 096, no. 012, 26. Dezember 1996 & JP 08 215162 A (OMRON CORP), 27. August 1996 & US 5 778 879 A
- PATENT ABSTRACTS OF JAPAN vol. 096, no. 012, 26. Dezember 1996 & JP 08 215160 A (OMRON CORP), 27. August 1996

## Beschreibung

Die vorliegende Erfindung betrifft ein Verfahren zur Bestimmung des Blutdrucks gemäß dem Oberbegriff des Anspruchs 1. Die vorliegende Erfindung betrifft ferner ein Blutdruckmeßgerät gemäß dem Oberbegriff des Anspruchs 6.

Blutdruckmessungen am Handgelenk oder an einem Finger leiden oftmals an mangelnder Meßgenauigkeit und unzureichender Reproduzierbarkeit. Dies ist durch die hohe Empfindlichkeit der Messung bezüglich Schwankungen der Meßposition bedingt, d. h. der individuellen Lage des Handgelenkes bzw. des Fingers, relativ zur Lage des Herzens. Um exakte Ergebnisse zu erhalten, ist bei bekannten Meßgeräten eine Messung auf Herzhöhe erforderlich. Dies wird jedoch in der Regel nur näherungsweise eingehalten und wird von den Personen, bei denen die Blutdruckmessung durchgeführt wird, als beschränkend und unpraktisch empfunden. Den Messungen wohnt deshalb stets eine Ungenauigkeit inne. Im Falle einer von der Herzhöhe abweichenden Meßposition verfälscht die hydrostatische Druckdifferenz das Meßergebnis um etwa 0,78 mm Hg/cm. Eine mangelhafte Lage während eines Meßvorganges führt somit zu einem systematischen Meßfehler. Zusätzlich kann eine kurzzeitige, eher zufällige Lageschwankung wie beispielsweise Zittern oder eine Armbewegung noch einen zweiten dynamischen Fehler, sog. Bewegungsartefakte, zur Folge haben, die die algorithmische Auswertung der eigentlichen Meßgröße erheblich erschweren, wenn nicht sogar unmöglich machen.

In der US-A 47 79 626 wurde bereits vorgeschlagen, bei einer Blutdruckmessung an einem Finger die hydrostatische Komponente des Blutdrucks mittels einer Vorrichtung zu kompensieren, die einen der hydrostatischen Druckkomponente entsprechenden Gegendruck bereitstellt. Hierzu wird auf Höhe des Herzens an der Brust ein Flüssigkeitsreservoir befestigt, das mittels eines Schlauches mit dem Blutdruckmeßgerät am Finger verbunden ist. Der Drucksensor des Meßgerätes ist dabei als Differenzdrucksensor ausgebildet, der die Druckdifferenz zwischen dem im Finger herrschenden Blutdruck und dem am Ende des Schlauches herrschenden Flüssigkeitsdruck mißt. Es liegt jedoch auf der Hand, daß dieses Blutdruckmeßgerät unhandlich und nur umständlich anzulegen ist. Darüber hinaus behindert der am Körper verlegte Schlauch die Bewegungsfreiheit der jeweiligen Person.

Die DE 296 12 412 U1 beschreibt ein Blutdruckmeßgerät, das an das Handgelenk anzulegen ist und bei dem im Gehäuse des Blutdruckmeßgerätes ein Pendel angeordnet ist. Die Außenseite des Pendels ist mit einer Farbskalierung versehen, so daß abhängig von der Armbeugenstellung eine bestimmte Farbe sichtbar wird. Hierbei ist die Handhabung u.a. insoweit erschwert, als daß das Pendel sich erst ausschwingen muß bis eine Ablesung möglich ist.

Aus der JP 08215161 ist ein Verfahren und Meßgerät zur Bestimmung des Blutdrucks mit einem Drucksensor und einer Ausrichtungs-Erfassungseinheit bestehend aus einem Neigungssensor bekannt. Die Ausrichtungs-Erfassungseinheit gibt ein elektrisches Signal zur Korrektur des erfaßten Blutdrucks entsprechend der Winkelstellung ab.

Aus der JP 08215162 ist ein Verfahren und ein Meßgerät zur Bestimmung des Blutdrucks mit einem Drucksensor und einer Ausrichtungs-Erfassungseinheit bestehend aus einem Neigungssensor ebenfalls bekannt. Die Ausrichtungs-Erfassungseinheit gibt ein elektrisches Signal ab, das eine Rückmeldung zur korrekten Meßposition gibt.

Der vorliegenden Erfindung liegt daher die Aufgabe zugrunde, ein Verfahren und ein Gerät zur Bestimmung des Blutdruckes zu schaffen, das bei hoher Meßgenauigkeit und ausreichender Reproduzierbarkeit der Blutdruckbestimmung einfach zu handhaben ist.

In verfahrenstechnischer Hinsicht wird diese Aufgabe erfindungsgemäß mit einem Verfahren mit den Merkmalen des Anspruchs 1 gelöst. Dies geschieht durch ein Verfahren zur Bestimmung des Blutdrucks, wobei ein Drucksensor an einem Körperglied angelegt und ein in dem Körperglied herrschender Blutdruck und die Ausrichtung des Körperglieds mit einer Ausrichtungs-Erfassungseinheit erfaßt wird, wobei die Ausrichtungs-Erfassungseinheit ein elektrisches Signal je nach der detektierten Ausrichtung des Körperglieds abgibt und dieses elektrische Signal weiterverarbeitet wird und wobei die Ausrichtung des Körperglieds mit der Ausrichtungs-Erfassungseinheit innerhalb eines Blutdruckmeßgerätegehäuses erfaßt wird und das elektrische Signal das von der Ausrichtungs-Erfassungseinheit abgegeben wird, zeitabhängig oder abhängig von einer Durchschaltung mittels einer Schaltweiche oder einem Multiplex-Verfahren zur Auswertung weitergeleitet wird.

Durch die elektrische Erfassung der Ausrichtung des Körpergliedes sind vielfältige Weiterverarbeitungsoptionen des elektrischen Signales möglich, die zu einer optimalen Handhabung und erhöhter Meßgenauigkeit der Blutdruckbestimmung führen. Das in der Ausrichtungs-Erfassungseinheit vorgesehene bewegliche Teil, daß z.B. pendelartig ausgebildet ist, weist eine bestimmte Schwingeigenfrequenz auf, die eine Periodendauer dei Schwingung festlegt. Abhängig von der Art der Bewegung des Patienten wird somit eine unterschiedlich starke Schwingungsamplitude in der Ausrichtungs-Erfassungseinhei erzeugt, die zunächst maximal anschwingt und eine gewisse Zeit zum ausschwinger benötigt. Durch die elektrische Weiterverarbeitung des Meßsignales ist es somit möglich dieses. Signal zu integrieren über die Zeit bzw. einen Mittelwert zu bilden über die Periodendauer, so daß das Signal stark gedämpft und damit letztlich erst ablesbar oder weiter nutzbar wird, da kleinere Ausschläge und ein kürzeres Nachschwingen des beweglichen Mittels in der Ausrichtungs-Erfassungseinheit erzeugt wird. Es wird somit ein Problem gelöst, daß vor allem bei einer sehr kompakten Ausrichtungs-Erfassungseinheit, die innerhalb eines Blutdruckmeßgerätegehäuses angeordnet ist auftreten kann. Weitere Optionen zur Weiterverarbeitung des elektrischen Signals werden nachfolgend aufgeführt.

Vorzugsweise wird der erfaßte Blutdruck in einer Auswerteeinheit entsprechend der erfaßten Ausrichtung des Körperglieds korrigiert. Bei einer vorgegebenen Ausrichtung des Körpers, insbesondere einer aufrechten Haltung des Oberkörpers, kann mit der Erfassungseinheit die absolute Ausrichtung des Körperglieds im Raum erfaßt und dadurch die Stellung des Körperglieds relativ zum Herzen bestimmt werden. Dementsprechend kann der tatsächlich gemessene Blutdruck im Körperglied in Abhängigkeit eines elektrischen Ausrichtungssignales der Erfassungseinheit korrigiert und der auf Herzhöhe herrschende Blutdruck bestimmt werden.

Gemäß einer bevorzugten Ausführung der Erfindung wird mit einem Neigungssensor die Winkelstellung des Körperglieds, insbesondere des Unterarmes, erfaßt und der erfaßte Blutdruck entsprechend der Winkelstellung korrigiert. Insbesondere kann die Neigung des Unterarmes zur Horizontalen bzw. Vertikalen erfaßt werden, die bei einer vorgegebenen Position des Ellenbogens, beispielsweise in einer am Oberkörper anliegenden Stellung, ein Maß für die Höhenlage des Handgelenkes und damit der hydrostatischen Komponente des Blutdrucks im Handgelenk ist.

Um eine einfache Signalverarbeitung zu ermöglichen, wird vorzugsweise die Ausrichtung des Körperglieds und der Blutdruck in demselben nacheinander erfaßt. Dieser ermöglicht es, bei ausreichender Genauigkeit, das Ausrichtungs- und Drucksignal nacheinander zu verarbeiten.

Gemäß einer anderen bevorzugten Ausführung der Erfindung wird die Ausrichtung des Körperglieds zeitparallel zur Druckmessung erfaßt. Dies erlaubt eine erhöhte Genauigkeit der Messung, da der Korrektur des erfaßten Blutdrucks stets die jeweilige Stellung des Körperglieds zugrunde gelegt werden kann.

In vorteilhafter Weiterbildung der Erfindung kann während der Druckerfassung eine Bewegung, insbesondere eine Beschleunigung des Körperglieds, an dem der Blutdruck gemessen wird, erfaßt werden. Der erfaßte Blutdruck wird dann entsprechend der erfaßten Bewegung korrigiert. Hierdurch können kurzzeitige Lageänderungen, wie z. B. Zittern während der Messung erfaßt werden. Durch eine Rückrechnung von den gemessenen Lageschwankungen auf die dazu korrespondierenden Druckschwankungen kann der Einfluß von Bewegungsartefakten verringert werden. Vorzugsweise wird die Ausrichtung und die Bewegung des Körperglieds kontinuierlich erfaßt.

Hinsichtlich des Gerätes wird die obengenannte Aufgabe mit einem Blutdruckmeßgerät erfindungsgemäß gelöst, das einen Drucksensor zur Erzeugung eines Drucksignals, eine Anlegeeinheit zum Anlegen des Drucksensors an ein Körperglied und eine Auswerteeinheit zur Auswertung des Drucksignals aufweist, wobei eine Ausrichtungs-Erfassungseinheit zur Erfassung der Ausrichtung des Körperglieds vorgesehen ist, durch die zur Weiterverarbeitung ein elektrisches Signal in Abhängigkeit von der Ausrichtung des Körperglieds abgebbar ist, wobei die Ausrichtungs-Erfassungseinheit innerhalb eines Blutdruckmeßgerätegehäuses angeordnet ist und wobei das von der Ausrichtungs-Erfassungseinheit abgebbare elektrische Signal über ein Zeitschaltglied oder einen Multiplexer oder eine Schaltweiche mit der Auswerteeinheit elektrische beschaltet ist.

Es können Schwingungen in der Ausrichtungs-Erfassungseinheit auf ein genau spezifiziertes Dämpfungsverhalten eingestellt werden ohne den Nachteil eine aufwendige mechanische Dämpfung, die zu dem fertigungsabhängig in ihrer Genauigkeit ist, vornehmen zu müssen.

Bevorzugt weist die Auswerteeinheit eine Korrektureinheit zur Korrektur des Drucksignales entsprechend der erfaßten Ausrichtung auf. Die Ausrichtungs-Erfassungseinheit erzeugt ein elektrisches Signal, das der Ausrichtung entspricht und das in der Korrektureinheit zur Korrektur des Drucksignales verarbeitet wird.

Die Ausrichtungs-Erfassungseinheit weist in vorteilhafter Weise einen Neigungssensor auf, der die Neigung des Körperglieds, an dem der Drucksensor angelegt ist, bzw. die Neigung der Anlegeeinheit, die der Neigung des Körperglieds entspricht, erfaßt. Vorzugsweise ist der Neigungssensor derart ausgebildet, daß er die absolute Neigung des Körperglieds, d. h. den Winkel zur Horizontalen bzw. Vertikalen erfaßt. Der Neigungswinkel gibt ein Maß für die Stellung des Körperglieds an.

Gemäß einer bevorzugten Ausführung der Erfindung kann eine Bewegungs-Erfassungseinheit zur Erfassung einer Bewegung, insbesondere einer Beschleunigung des Körperglieds vorgesehen sein, wobei ein elektrisches Bewegungssignal in der Auswerteeinheit verarbeitet wird. Durch das Bewegungssignal ist das Maß an motorischer Aktivität der jeweiligen Person feststellbar. Auf diese Weise läßt sich beurteilen, ob sich der Anwender generell in einem Zustand ausreichender Ruhe befindet und eine Messung überhaupt sinnvoll durchführbar ist.

Dabei kann die Messung dann, wenn ein zu ungenaues Ergebnis geliefert werden würde, unterbunden werden oder zumindest ein Hinweis auf die geringe Aussagekraft der Messung auf einer Anzeigevorrichtung angezeigt werden. Vorteilhafterweise kann das erfaßte Drucksignal in Abhängigkeit des elektrischen Bewegungssignales in der Korrektureinheit der Auswerteeinheit korrigiert werden.

Vorzugsweise umfaßt die Bewegungs-Erfassungseinheit den Neigungssensor und eine mit diesem verbundene Differenziereinheit, die die zeitliche erste oder zweite Ableitung des Neigungssignales bildet und das errechnete Signal als Bewegungssignal bereitstellt.

Entsprechend einer bevorzugten Ausführung der Erfindung sind die Ausrichtungs-Erfassungseinheit und der Drucksensor mit der Auswerteeinheit über eine Zeitschalteinheit zur zeitabhängigen Durchschaltung des jeweiligen Signales an die Auswerteeinheit oder über eine Schaltweiche bzw. einen Multiplexer verbunden.

Zwischen die Schalteinheit und die Auswerteeinheit ist zumindest ein Analog-Digital-Wandler geschaltet. Um die Ausrichtung des Körperglieds zeitparallel zur Druckmessung erfassen zu können, sind bevorzugt zwei Analog-Digital-Wandler vorgesehen.

Zweckmäßigerweise weist das Blutdruckmeßgerät eine Speichereinheit zur Speicherung von Referenzdaten auf. Mit Hilfe der Referenzdaten können die Korrekturwerte für das Drucksignal an den jeweiligen Benutzer angepaßt werden. Beispielsweise können für Benutzer unterschiedlicher Körpergröße verschiedene Referenzdatensätze abgespeichert sein.

Die Druckmessung kann grundsätzlich an verschiedenen Körpergliedern, beispielsweise an einem Fingern, vorgenommen werden. Vorzugsweise jedoch ist die Anlegeeinheit zum Anlegen des Drucksensors an ein Handgelenk ausgebildet. Die Druckerfassung am Handgelenk erlaubt bei einer einfach Erfassung der Ausrichtung eine hohe Meßgenauigkeit.

Nachfolgend wird die vorliegende Erfindung anhand von Ausführungsbeispielen und zugehöriger Zeichnungen näher erläutert. In diesen zeigen:
- Figur 1: ein Blutdruckmeßgerät entsprechend einer bevorzugten Ausführung der Erfindung, das an einem Handgelenk einer Person angelegt ist, in einer schematischen Darstellung,
- Figur 2: den Aufbau eines Blutdruckmeßgerätes entsprechend einer bevorzugten Ausführung der Erfindung in einer schematischen Darstellung,
- Figur 3: ein Flußdiagramm, das die einzelnen Schritte eines Verfahrens zur Bestimmung des Blutdrucks gemäß einer bevorzugten Ausführung der Erfindung zeigt, daß mit der Vorrichtung gemäß Figur 2 ausführbar ist,
- Figur 4: den Aufbau eines Blutdruckmeßgerätes gemäß einer weiteren Ausführung der Erfindung in einer Darstellung ähnlich Figur 2, und
- Figur 5: ein Flußdiagramm ähnlich Figur 3, das die Schritte eines Verfahrens zur Bestimmung des Blutdrucks entsprechend einer weiteren Ausführung der Erfindung zeigt, das mit Hilfe des Blutdruckmeßgerätes gemäß Figur 4 ausführbar ist.

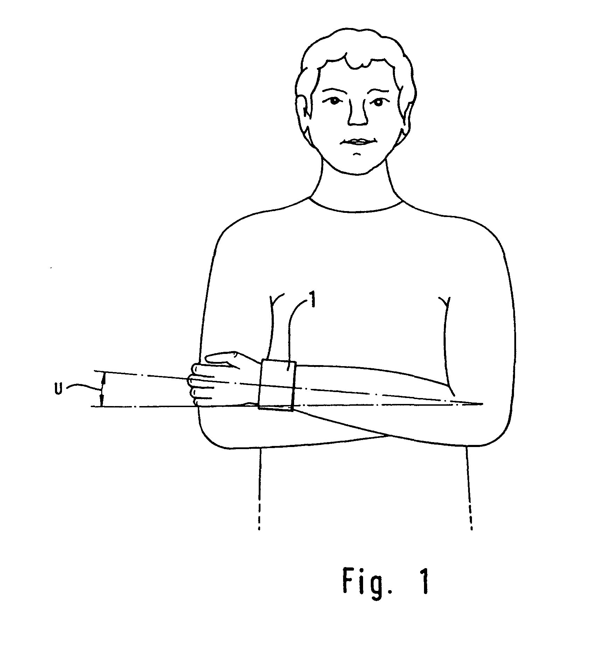

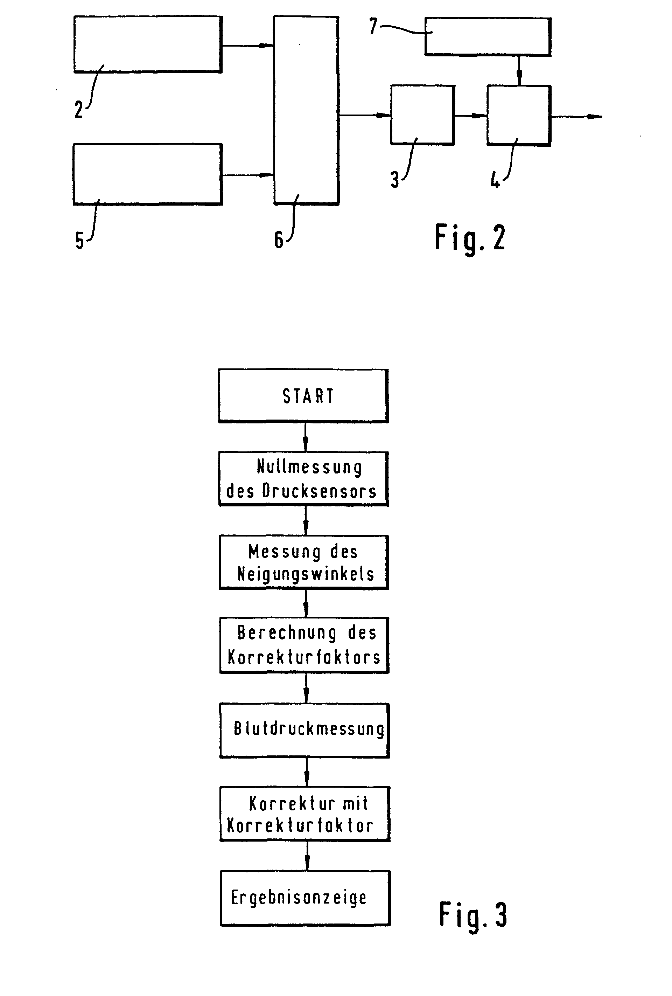

Das in Figur 1 gezeigte Blutdruckmeßgerät ist zur Messung des Blutdrucks am Handgelenk ausgebildet. Es weist als Anlegeeinheit eine Manschette 1 auf, mit der ein Drucksensor zur Signalaufnahme an die Innenseite des linken Handgelenkes angelegt werden kann. Die Manschette 1 weist eine Blase auf, die vorzugsweise mit Luft aufgepumpt wird, um während des Luftablassens mittels der oszillometrischen Meßmethode den diastolischen, den systolischen, eventuell den mittleren Blutdruck- und den Puls zu bestimmen. Der Drucksensor 2 (Figur 2) kann z.B. als kapazitiver oder piezoresistiver Sensor ausgebildet sein.

Der Drucksensor 2 ist über einen Verstärker und Analog/Digital-Wandler 3 mit einer Auswerteeinheit 4 verbunden, die als Microcontroller ausgebildet und zur algorithmischen Auswertung des elektrischen Signales des Drucksensors ausgebildet ist.

Das Blutdruckmeßgerät weist ferner einen Neigungssensor 5 auf, der ebenfalls über den Verstärker und Analog/Digital-Wandler 3 mit der Auswerteeinheit 4 verbunden ist. Der Neigungssensor 5 erfaßt die Neigung der Manschette 1 und damit des Handgelenks bzw. des Unterarmes gegenüber der Horizontalen, d. h. der Neigungssensor 5 stellt ein elektrisches Signal bereit, das dem Neigungswinkel u des Handgelenks zur Horizontalen entspricht (vgl. Figur 1). Der Neigungssensor weist vorzugsweise ein beweglich gelagertes Teil, wie z.B. ein Pendel auf und ist mit einer Einrichtung versehen, mit der der Neigungswinkel u, die Geschwindigkeit oder die Beschleunigung des beweglichen Teils elektrisch erfaßbar ist. Je nach dem für welchen Zweck das detektierte Signal verwendet werden soll, um z.B. verschiedene Arten von Bewegungsartifakte zu unterscheiden, wird auf dem Neigungswinkel, die Geschwindigkeit oder die Beschleunigung des beweglichen Teils zurückgegriffen. Die Geschwindigkeit und die Beschleunigung des beweglichen Teils im Neigungssensor kann z.B. durch die elektronische Erzeugung der ersten und zweiten Ableitung oder durch speziell dafür ausgebildete Sensoren detektiert werden, um z.B. verschiedene Arten von Bewegungsartefakten zu unterscheiden und entsprechend zu berücksichtigen.

Wie Figur 2 zeigt, sind der Drucksensor 2 und der Neigungssensor 5 mit dem gemeinsamen Verstärker und Analog/Digital-Wandler 3 über ein Zeitschaltglied 6, das als Multiplexer ausgebildet ist, verbunden. Durch den Multiplexer 6 wird wahlweise das Signal des Drucksensors 2 oder das Signal des Neigungssensors 5 auf den Verstärker und Analog/Digital-Wandler 3 durchgeschaltet. Die in Figur 2 skizzierte Schaltung zur Signalaufbereitung ist vorzugsweise in einem Schaltkreis integriert, der abhängig von der Schaltstellung des Multiplexers 6 auch die Konfiguration des Verstärkers und Analog/Digital-Wandlers 3 den vom Sensor herrührenden Anforderungen anpaßt.

Um das Blutdruckmeßgerät an verschiedene Randbedingungen, insbesondere verschiedene Benutzer anpassen zu können, ist ein Referenzwertspeicher 7 vorgesehen, der mit der Auswerteeinheit 4 verbunden oder in ihr enthalten ist. Die individuellen Randbedingungen der Messung, die die Positionierung des Blutdruckmeßgerätes und damit das Meßergebnis beeinflussen, wie beispielsweise die Armlänge oder die Lage des Herzens, sind benutzerspezifisch verschieden. Es wird deshalb einmal eine Referenzmessung auf Herzniveau durchgeführt, deren Ergebnisse, insbesondere der dabei erfaßte Neigungswinkel u_{ref} und die von diesem Winkel abhängige Signalspannung, zusammen mit eventuellen Kalibrierdaten des Neigungssensors 5 im Referenzspeicher 7 abgelegt werden und bei allen nachfolgenden Blutdruckmessungen zur Korrektur dienen. Die Korrektur des jeweils gemessenen Blutdrucks ergibt sich dabei aus dem gemessenen Neigungswinkel u wie folgt:

Pₖₒᵣᵣ = Pₘₑₛₛ - k (1 - sin u/sin u_{ref}), wobei Pₖₒᵣᵣ und Pₘₑₛₛ die korrigierten bzw. gemessenen Druckwerte, u den gegenüber der Horizontallage des Blutdruckmeßgerätes gemessenen Winkel und u_{ref} den einmalig, gleichfalls gegen die Horizontallage bestimmten Referenzwinkel bei einer Lage des Geräts auf Herzniveau darstellen. Der Multiplikator k ist ein für die Kalibration erforderlicher konstanter Koeffizient. Entsprechend dieser Beziehung kann der tatsächlich gemessene Druck um den hydrostatischen Differenzdruck korrigiert und der auf der Herzhöhe zugehörige Blutdruck bestimmt werden. Ein lagebedingter systematischer Meßfehler wird dadurch vermieden.

Nachfolgend wird der Ablauf der Verfahrensschritte bei einer Blutdruckbestimmung in Verbindung mit Figur 3 näher erläutert. Zunächst wird vor der eigentlichen Messung eine Nullmessung des Drucksensors durchgeführt, um das Signalniveau des Drucksensors bei fehlendem Druck jedes Mal neu festzulegen und die Genauigkeit der Messung zu erhöhen. Sobald das Blutdruckmeßgerät mittels einer Manschette 1 am linken Handgelenk des Benutzers angelegt ist, bringt der Benutzer zur Blutdruckmessung die Arme in eine im wesentlichen vor dem Oberkörper verschränkte Position und seinen Oberkörper in eine im wesentlichen aufrechte Stellung (vgl. Figur 1). Dies stellt sicher, daß der erfaßte Neigungswinkel u in Korrelation steht mit der Lage des Handgelenkes relativ zum Herzen, d. h. daß der Neigungswinkel u ein Maß, für die Höhendifferenz zwischen der Lage des Handgelenkes und der Lage des Herzens ist. Um eine korrekte Meßstellung zu gewährleisten, ist eine Anzeigevorrichtung des Blutdruckmeßgerätes derart angeordnet, daß diese vom Benutzer nur in einer korrekten Stellung ablesbar ist. Die Anzeigevorrichtung ist an der oberen Schmalseite der Manschette 1, d. h. in dem Bereich, der im angelegten Zustand an der oberen Schmalseite des Gelenkes etwa in Fortsetzung des Daumengliedes liegt angeordnet.

Hat der Benutzer die entsprechende Stellung eingenommen, wird zunächst mit dem Neigungssensor 5 der Neigungswinkel u des Blutdruckmeßgerätes bestimmt. Das Zeitschaltglied 6 schaltet das Signal des Neigungssensors 5 zu dem Verstärker und Analog/DigitalWandler 3 durch, so daß dieses von der Auswerteeinheit 4 ausgewertet werden kann. Dabei wird in einem nächsten Verfahrensschritt der Korrekturfaktor bestimmt, um den der tatsächlich gemessene Blutdruck dann zu korrigieren ist. Der im Handgelenk herrschende Blutdruck wird dann in einem weiteren Verfahrensschritt bestimmt. Hierzu wird das Signal des Drucksensors 2 von dem Zeitschaltglied 6, zur Auswerteeinheit durchgeschaltet. Anschließend wird der tatsächlich erfaßte Blutdruck um den zuvor bestimmten Korrekturwert korrigiert. Das korrigierte Meßergebnis des Blutdrucks wird dann an der Anzeigevorrichtung des Blutdruckmeßgerätes angezeigt.

Alternativ kann die Vermeidung lagebedingter Fehlmessungen auch über die Anzeigevorrichtung des Blutdruckmeßgerätes interaktiv mit dem Benutzer gesteuert werden. Hierzu erhält der Anwender solange Anzeichen auf der Anzeigevorrichtung des Blutdruckmeßgerät bis er es innerhalb eines vorgegebenen Winkeltoleranzfeldes um den Referenzwinkel gebracht hat und damit eine Meßposition eingenommen hat, in der keine Korrektur des Blutdruckmeßwertes mehr erforderlich ist, oder eine Korrektur durchgeführt werden muß. In einer weiteren Ausführungsform wird das Anzeichen auf der Anzeigevorrichtung zur Benutzerführung für die optimale Meßlage am Anfang und/oder während der Druckmessung eingesetzt. Beispielsweise weist die Anzeigevorrichtung als Anzeichen einen nach oben und nach unten weisenden Pfeil auf, von denen nur der gerade angezeigt wird oder blinkt, wenn der Benutzer sein Handgelenk mit dem Blutdruckmeßgerät entsprechend nach oben oder unten bewegen soll, um in die korrekte Meßposition zu gelangen. Eine Benutzerführung durch eine rote Lampe für eine schlechte Meßposition und /-oder eine grün aufleuchtende Lampe für eine korrekte Meßposition oder auch durch ein akustisches Warnsignal bei einer schlechten Meßposition ist alternativ ebenso als Anzeichen ausbildbar. Vorzugsweise wird das Anzeigesignal für die korrekte/unkorrekte Meßposition nur vor der Messung an der elektronischen Anzeigevorrichtung (z.B. LCD) angezeigt. Alternativ können auch andere Zeitpunkte (z.B. auch während der Messung, z.B. für den Fall einer Veränderung der Meßposition) zur Anzeige programmiert werden. Dadurch, daß der Neigungssensor ein elektrisches Signal abgibt, ist es ohne weiteres möglich, abweichend von einer ständigen evtl. den Benutzer irritierenden Anzeige der Meßposition diese nur zu bestimmten Zeitpunkten elektronisch anzuzeigen.

Umgekehrt wird in einer weiteren Ausführungsform in einem Meßwertspeicher eine konkrete Validierung der Meßergebnisse hinsichtlich der detektierten Meßlage durchgeführt. Somit wird beispielsweise nach der Messung angezeigt, ob das Meßergebnis wegen einer ungünstigen Meßlage oder einer Bewegung während der Messung verworfen werden sollte oder ob eine Kompensierung bzw. Korrektur bzw. Eliminierung von lagebedingt fehlerhaften Meßwerten erfolgt ist.

Die in den obigen beiden Absätzen dargestellten Ausführungsformen sind sowohl in Kombination mit der ersten als auch mit der nachfolgend beschriebenen zweiten Ausführungsform kombinierbar.

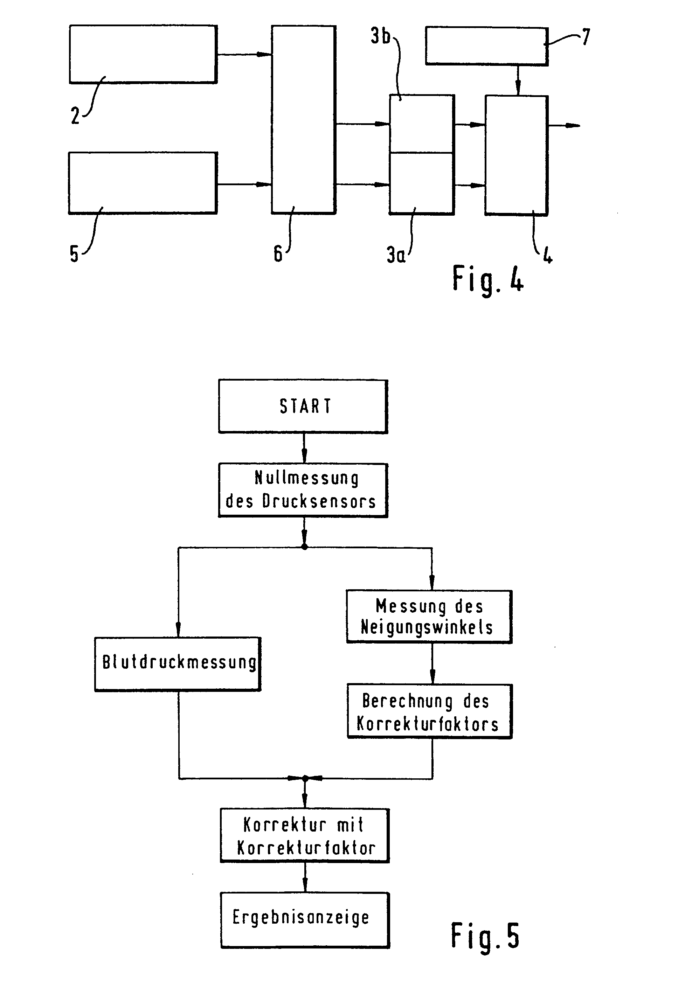

Eine zweite Ausführung eines erfindungsgemäßen Blutdruckmeßgerätes ist in Figur 4 dargestellt. Der Aufbau des Blutdruckmeßgerätes ist grundsätzlich ähnlich dem in Figur 2 gezeigten, so daß für dieselben Bauteile gleiche Bezugszeichen verwendet sind und eine nochmalige Beschreibung derselben Bauteile nicht notwendig ist. Die Ausführung gemäß Figur 4 unterscheidet sich im wesentlichen dadurch, daß der Drucksensor 2 und der Neigungssensor 5 nicht über einen gemeinsamen Verstärker und Analog/Digital-Wandler mit der Auswerteeinheit 4 verbunden sind, sondern daß sowohl der Neigungssensor 5 als auch der Drucksensor 2 jeweils separat über einen eigenen Verstärker und Analog/Digital-Wandler 3 a bzw. 3 b mit der Auswerteeinheit 4 verbunden sind. Hierdurch kann das Signal des Neigungssensors 5 kontinuierlich der Auswerteeinheit 4 bereitgestellt werden. Der Neigungswinkel u wird zeitparallel zur Erfassung des Blutdrucks im Handgelenk erfaßt.

Zur Erfassung einer Bewegung bzw. Beschleunigung des Handgelenkes weist die Auswerteeinheit 4 eine Differenziereinheit auf, mit Hilfe derer die zeitliche Ableitung des Signals des Neigungssensors 5, die dann der Bewegung bzw. (Winkel-) Geschwindigkeit entspricht, und die Ableitung der zeitlichen Ableitung, die dann der (Winkel-) Beschleunigung entspricht, bestimmbar ist.

Der Ablauf der Verfahrensschritte des Verfahrens zur Blutdruckbestimmung, das mit der Vorrichtung gemäß Figur 4 ausführbar ist, ist in Figur 5 dargestellt. Wie in Figur 5 zu sehen ist, wird der Neigungswinkel zeitparallel zur Blutdruckmessung bestimmt und zur Berechnung des Korrekturfaktors verwendet.

Aus der bestimmten Bewegung (Geschwindigkeit) bzw. Beschleunigung des Handgelenkes wird zunächst festgestellt, ob sich der Anwender generell in einem Zustand ausreichender Ruhe befindet und eine Messung sinnvoll durchführbar ist. Wird ein erhöhtes Maß an motorischer Aktivität festgestellt, so daß nur ein unrepräsentatives Blutdruckmeßergebnis geliefert werden kann, wird die Messung unterbunden bzw. auf der Anzeigevorrichtung ein Hinweis auf die geringe Aussagekraft der Messung angezeigt. Diese Ausbildung des Blutdruckmeßgerätes und dieses Verfahren der Anzeige aufgrund geringer Aussagekraft der Messung in Folge einer ungeeigneten Winkelstellung des Körpergliedes, an dem das Blutdruckmeßgerät befestigt ist oder aufgrund von bestimmten detektierten Bewegungen, die das Meßergebnis stark verfälschen würden, ist unabhängig von den übrigen Korrekturund Anzeigemöglichkeiten oder in beliebiger Kombination durchführbar. Die Unterbindung der Messung bzw. der Hinweis auf geringe Aussagefähigkeit der Messung kann vor und oder während der Messung oder nach der Messung durchgeführt werden.

Darüber hinaus wird der gemessene. Blutdruck mit Hilfe der erfaßten Bewegung (Geschwindigkeit) und Beschleunigung korrigiert. Kurzzeitige Lageänderungen wie beispielsweise Kurzzeitbewegungen oder Zittern des Handgelenkes während der Blutdruckmessung machen sich in erster Linie durch Schwankungen im Ausgangssignal des Drucksensors bemerkbar. Durch eine Rückrechnung von den gemessenen Lageschwankungen auf die dazu korrespondierende Druckschwankung kann der Einfluß der Bewegungsartefakte verringert werden.

Das durch den Neigungssensor 5 erzeugte Meßsignal wird vorzugsweise elektronisch nachgearbeitet, so daß ein gedämpftes Schwingungsverhalten des beweglichen Teils im Neigungssensor mit kleineren schwingungsamplituden und einem kürzeren Ausschwingen als mechanisch generiert elektronisch durch Filterung, Integration, bzw. Mittelung über die Periodendauer des beweglichen Teils erzeugt wird. Gegenüber mechanischen Dämpfungssystemen ist dadurch ein optimales Dämpfungsverhalten (auch ein nicht lineares) unabhängig von der Fertigungsqualität des beweglichen Teils einstellbar. Dies ermöglicht auch eine wesentlich bessere Ablesbarkeit des gemessenen Signals an der Anzeigevorrichtung.

Die Anzeigevorrichtung auf der das aufbereitete Meßergebnis des Neigungssensors angezeigt wird ist dieselbe, mit der die Blutdruckmeßwerte und gegebenenfalls der Puls angezeigt wird. Es handelt sich dabei um eine elektronische Anzeigeeinrichtung vom Typ z.B. eines LCD, ST, STN, TFT oder ähnlichen Displays. Es sind grundsätzlich z.B. alle Displaytypen verwendbar, die auch für tragbare Computeranzeigevorrichtungen eingesetzt werden. Nachdem bekanntermaßen die Ablesbarkeit von Anzeigen auf diesen Anzeigevorrichtungen vom Betrachtungswinkel relativ zur Anzeigevorrichtung abhängt, kann in einer weiteren Ausführungsform das Meßergebnis des Neigungssensors ergänzend oder ausschließlich dafür verwendet werden, daß in einer Anpassungseinrichtung die elektronische Anzeigeeinrichtung an den Blickwinkel angepaßt wird, da in der Regel ein Zusammenhang zwischen den möglichen Neigungswinkeln am Handgelenk und den möglichen Blickwinkeln relativ zum Blutdruckmeßgerät mit Anzeigevorrichtung am Handgelenk besteht.

Das Ergebnis der beschriebenen Nullmessung des Drucksensors 2 kann auch in dem Referenzwertspeicher 7 abgelegt werden, so daß je Bedienperson nur insgesamt einmal eine spezifische Kalibrierung stattfinden muß und später nur noch die Bedienperson einzugeben ist.

Unter dem Begriff Drucksensor im Sinne der vorliegenden Anmeldung sind allgemein Sensoren zu verstehen, die ein Drucksignal bereitstellen, aus dem sich der in dem Körperglied herrschende Blutdruck bestimmen läßt. Dies kann beispielsweise auch ein optischer Sensor, wie beispielsweise ein Infrarotsensor sein, der den Puls optisch erfassen kann, so daß sich aus der zeitlichen Veränderung des Signales der Manschetteninnendruck detektieren läßt.

## Patentansprüche

1. Verfahren zur Bestimmung des Blutdrucks am Unterarm, wobei eine Anlegeeinheit (1) mit einem Drucksensor und einer Anzeigevorrichtung am Handgelenk angelegt und die Ausrichtung des Unterarms mit einer Ausrichtungs-Erfassungseinheit (5) erfaßt wird, wobei die Ausrichtungs-Erfassungseinheit (5) ein elektrisches Signal je nach der detektierten Ausrichtung des Unterarms abgibt und dieses elektrische Signal weiterver-arbeitet wird, wobei ein in dem Unterarm herrschender Blutdruck gemessen wird wobei die Ausrichtung des Unterarms mit der Ausrichtungs-Erfassungseinheit (5) innerhalb eines Blutdruckmeßgerätegehäuses erfaßt wird und **dadurch gekennzeichnet, daß** die Anzeigevorrichtung des Blutdruckmeßgerätes an der oberen Schmalseite der Anlegeeinheit (1), zur Positionierung der Anzeigevorrichtung an der oberen Schmalseite des Handgelenks in Fortsetzung des Daumengliedes, angeordnet wird.

2. Verfahren nach Anspruch 1, **dadurch gekennzeichnet, daß** der erfaßte Blutdruck in einer Auswerteeinheit (4) entsprechend der erfaßten Ausrichtung des Unterarms korrigiert wird.

3. Verfahren nach Anspruch 1, **dadurch gekennzeichnet, daß** die Winkelstellung (u) des Unterarmes, mit einem Neigungssensor (5) erfaßt und der erfaßte Blutdruck entsprechend der Winkelstellung korrigiert wird.

4. Verfahren nach Anspruch 1 oder 2, **dadurch gekennzeichnet, daß** eine Bewegung, insbesondere Geschwindigkeit oder Beschleunigung, des Unterarms während der Druckerfassung erfaßt und der erfaßte Blutdruck entsprechend der Bewegung, insbesondere Geschwindigkeit oder Beschleunigung, des Unterarms korrigiert wird.

5. Verfahren nach zumindest einem der vorhergehenden Ansprüche, **dadurch gekennzeichnet, daß** über die Anzeigevorrichtung anwenderinteraktiv ein Anzeichen eine Rückmeldung ausgibt, ob die Meßposition in einem korrekten Winkelbereich des Unterarms an dem die Messung erfolgt liegt und / oder das Anzeichen den Anwender interaktiv zur korrekten Meßposition führt.

6. Verfahren nach Anspruch 5, **dadurch gekennzeichnet, daß** als Anzeichen nach oben und nach unten weisende Pfeile vorgesehen sind.

7. Verfahren nach Anspruch 6, **dadurch gekennzeichnet, daß** von den Pfeilen gerade der angezeigt wird oder blinkt, abhängig ob der Benutzer sein Handgelenk mit dem Blutdruckmeßgerät entsprechend nach oben oder nach unten bewegen soll, um in eine korrekte Meßposition zu gelangen.

8. Verfahren nach einem der Ansprüche 5 bis 7 **dadurch gekennzeichnet, daß** das Anzeichen nur zu bestimmten Zeitpunkten elektronisch angezeigt wird.

9. Verfahren nach Anspruch 8 **dadurch gekennzeichnet, daß** bei geringer Aussagekraft der Blutdruckmessung in Folge einer ungeeigneten Winkelstellung des Unterarms, an dem das Blutdruckmeßgerät befestigt ist die Blutdruckmessung unterbunden wird, wobei die Unterbindung vor oder während der Blutdruckmessung erfolgt.

10. Verfahren nach einem der Ansprüche 1 bis 9, **dadurch gekennzeichnet, daß** die Ausrichtung des Unterarms zeitparallel zur Erfassung des Blutdrucks erfolgt.

11. Verfahren nach einem der Ansprüche 1 bis 10 **dadurch gekennzeichnet, daß** die Anzeigevorrichtung auf der das Meßergebnis der Ausrichtungs-Erfassungseinheit (5) angezeigt wird, dieselbe ist, mit der die Blutdruckmeßwerte und ein Puls angezeigt wird.

12. Blutdruckmeßgerät mit einem Drucksensor zur Erzeugung eines Drucksignals, einer Anlegeeinheit zum Anlegen des Drucksensors an ein Handgelenk eines Unterarms und einer Auswerteeinheit (4) zur Auswertung des Drucksignals, wobei eine Ausrichtungs-Erfassungseinheit (5) zur Erfassung der Ausrichtung des Unterarms vorgesehen ist, durch die zur Weiterverarbeitung ein elektrisches Signal in Abhängigkeit von der Ausrichtung des Unterarms abgebbar ist wobei die Ausrichtungs-Erfassungseinheit (5) innerhalb eines Blutdruckmeßgerätegehäuses angeordnet ist und **dadurch gekennzeichnet daß** die Anzeigevorrichtung an der Schmalseite der Anlegeeinheit (1) zur Positionierung der Anzeigevorrichtung an der oberen Schmalseite des Handgelenks des Unterarms in Fortsetzung des Daumengliedes, angeordnet ist.

13. Blutdruckmeßgerät nach Anspruch 12, **dadurch gekennzeichnet, daß** die Auswerteeinheit (4) eine Korrektureinheit zur Korrektur des Drucksignales entsprechend der erfaßten Ausrichtung aufweist.

14. Blutdruckmeßgerät nach Anspruch 12, **dadurch gekennzeichnet, daß** die Ausrichtungs-Erfassungseinheit einen Neigungssensor (5) aufweist, der die Neigung des Unterarms an dem der Drucksensor (2) angelegt ist, erfaßt.

15. Blutdruckmeßgerät nach Anspruch 12, 13 oder 14, **dadurch gekennzeichnet, daß** eine Bewegungs-Erfassungseinheit (5) zur Erfassung einer Bewegung, insbesondere einer Geschwindigkeit oder Beschleunigung, des Unterarms vorgesehen ist und die Auswerteeinheit (4) eine Korrektureinheit zur Korrektur des Drucksignales entsprechend der erfaßten Bewegung, insbesondere Geschwindigkeit oder Beschleunigung, aufweist.

16. Blutdruckmeßgerät nach Anspruch 15, **dadurch gekennzeichnet, daß** die Bewegungs-Erfassungseinheit den Neigungssensor (5) und eine mit diesem verbundene Differenziereinheit umfaßt.

17. Blutdruckmeßgerät nach zumindest einem der Ansprüche 12 bis 16, **dadurch gekennzeichnet, daß** die Ausrichtungs-Erfassungseinheit (5) und der Drucksensor (2) mit der Auswerteeinheit (4) über eine Zeitschalteinheit (6) verbunden sind.

18. Blutdruckmeßgerät nach der Anspruche 17, **dadurch gekennzeichnet, daß** die Zeitschalteinheit (6) als Schaltweiche oder Multiplexer ausgebildet ist.

19. Blutdruckmeßgerät nach zumindest einem der Ansprüche 12 bis 16, **dadurch gekennzeichnet, daß** die Ausrichtungs-Erfassungseinheit (5) und der Drucksensor (2) jeweils separat mit der Auswerteeinheit (4) verbunden sind, so daß die Ausrichtung des Unterarms zeitparallel zur Erfassung des Blutdrucks erfolgt.

20. Blutdruckmeßgerät nach einem der Ansprüche 12 bis 19, **dadurch gekennzeichnet, daß** eine Speichereinheit (7) zur Speicherung von Referenzdaten vorgesehen ist,

21. Blutdruckmeßgerät nach einem der Ansprüche 12 bis 20, **dadurch gekennzeichnet, daß** eine Anzeigevorrichtung vorgesehen ist, die Anzeichen aufweist, die derart angezeigt werden, daß ein korrekter und/oder unkorrekter Winkelbereich oder eine Bewegung des Blutdruckmeßgeräts und/oder ein Hinweis zur korrekteren Meßlage ausgegeben werden.

22. Blutdruckmeßgerät nach Anspruch 21 **dadurch gekennzeichnet, daß** als Anzeichen zwei in entgegengesetzte Richtung zeigende Pfeile vorgesehen sind.

23. Blutdruckmeßgerät nach Anspruch 21 oder 22 **dadurch gekennzeichnet, daß** das Anzeichen nur zu bestimmten Zeitpunkten, insbesondere vor oder während der Blutdruckmessung, elektronisch anzeigbar ist.

24. Blutdruckmeßgerät nach einem der Ansprüche 12 bis 23, **dadurch gekennzeichnet, daß** ein Meßwertspeicher und oder eine Einrichtung aufweist zur Validierungsbestimmung der Meßergebnisse, so daß abhängig von der Meßlage, dem Meßneigungswinkel, oder der Bewegung während der Messung ein Hinweis über die fehlerhaften Meßbedingungen ausgebbar ist.

25. Blutdruckmeßgerät nach einem der Ansprüche 12 bis 24 **dadurch gekennzeichnet, daß** die Anzeigevorrichtung auf der das Meßergebnis der Ausrichtungs-Erfassungseinheit (5) anzeigbar ist, dieselbe ist, mit der die Blutdruckmeßwerte und ein Puls anzeigbar sind.

## Claims

1. A method of determining the blood pressure on an individual's forearm, in which an application unit (1) comprising a pressure sensor and a display device is applied to an individual's wrist and the orientation of said forearm is detected by means of an orientation sensing unit (5), said orientation sensing unit (5) delivering an electrical signal responsive to the detected orientation of the forearm, and said electrical signal being further processed, with a blood pressure prevailing in the forearm being measured, and the forearm's orientation being detected with the orientation sensing unit (5) in the interior of a housing of a blood pressure measuring device, **characterized in that** the display device of the blood pressure measuring device is arranged on the upper narrow side of the application unit (1) to position the display device on the upper narrow side of the wrist in extension of the thumb.

2. The method as claimed in claim 1,
**characterized in that** the detected blood pressure is corrected in an evaluating unit (4) in response to the detected orientation of the individual's forearm.

3. The method as claimed in claim 1,
**characterized in that** the angular position (u) of the individual's forearm is detected by means of an inclination sensor (5), and the detected blood pressure is corrected in response to said angular position.

4. The method as claimed in claim 1 or 2,
**characterized in that** a motion, in particular speed or acceleration, of the individual's forearm is detected while the pressure is being sensed, and the detected blood pressure is corrected in response to said motion, in particular speed or acceleration of said forearm.

5. The method as claimed in at least one of the preceding claims,
**characterized in that** a readout provided via the display device by user interaction provides a feedback indicative of whether the measurement position is in a correct angular range of the forearm from which the measurement is taken, and/or the readout causes the user, by interaction, to adopt the correct position for measurement.

6. The method as claimed in claim 5,
**characterized in that** for readout provision is made for up and down pointing arrows.

7. The method as claimed in claim 6,
**characterized in that** that particular one of the arrows is displayed or flashes that indicates that the user is required to move his wrist with the blood pressure measuring device up or down correspondingly to reach a proper position for measurement.

8. The method as claimed in any one of the claims 5 to 7,
**characterized in that** the readout is provided electronically only at predetermined instants of time.

9. The method as claimed in claim 8,
**characterized in that** in the presence of little informative value of the blood pressure measurement due to an unsuitable angular position of the forearm to which the blood pressure measuring device is attached the blood pressure measurement is stopped, such stopping taking place prior to or during the blood pressure measurement cycle.

10. The method as claimed in any one of the claims 1 to 9,
**characterized in that** the orientation of the forearm takes place simultaneously with the detection of the blood pressure.

11. The method as claimed in any one of the claims 1 to 10,
**characterized in that** the display device indicating the measurement result of the orientation sensing unit (5) is the same device that indicates the blood pressure measurement values and a pulse rate.

12. A blood pressure measuring device comprising a pressure sensor for generating a pressure signal, an application unit for applying the pressure sensor to a wrist of an individual's forearm, and an evaluating unit (4) for evaluating the pressure signal, with provision being made for an orientation sensing unit (5) for detecting the forearm's orientation and by means of which an electrical signal responsive to the forearm's orientation is deliverable for further processing, said orientation sensing unit (5) being arranged in the interior of a housing of the blood pressure measuring device,
**characterized in that** the display device is arranged on the narrow side of the application unit (1) to position the display device on the upper narrow side of the forearm's wrist in extension of the thumb.

13. The blood pressure measuring device as claimed in claim 12,
**characterized in that** the evaluating unit (4) includes a correcting unit for correcting the pressure signal in response to the detected orientation.

14. The blood pressure measuring device as claimed in claim 12,
**characterized in that** the orientation sensing unit includes an inclination sensor (5) that detects the inclination of the individual's forearm to which the pressure sensor (2) is applied.

15. The blood pressure measuring device as claimed in claim 12, 13 or 14,
**characterized in that** provision is made for a motion sensing unit (5) for detecting a motion, in particular a speed or acceleration of the individual's forearm, and said evaluating unit (4) includes a correcting unit for correcting the pressure signal in response to the detected motion, in particular the speed or acceleration.

16. The blood pressure measuring device as claimed in claim 15,
**characterized in that** said motion sensing unit comprises the inclination sensor (5) and a differentiating unit connected thereto.

17. The blood pressure measuring device as claimed in any one of the claims 12 to 16,
**characterized in that** the orientation sensing unit (5) and the pressure sensor (2) are connected to the evaluating unit (4) via a timing unit (6).

18. The blood pressure measuring device as claimed in claim 17,
**characterized in that** said timing unit (6) is constructed as a switching device or multiplexer.

19. The blood pressure measuring device as claimed in at least one of the claims 12 to 16,
**characterized in that** the orientation sensing unit (5) and the pressure sensor (2) each are separately connected with the evaluating unit (4) so that the orientation of the forearm takes place simultaneously with detection of the blood pressure.

20. The blood pressure measuring device as claimed in any one of the claims 12 to 19,
**characterized in that** provision is made for a storage unit (7) for the storage of reference data.

21. The blood pressure measuring device as claimed in any one of the claims 12 to 20,
**characterized in that** provision is made for a display device providing a readout in such fashion that a correct and/or incorrect angular range or a movement of the blood pressure measuring device and/or a prompt for correcting the measurement position are output.

22. The blood pressure measuring device as claimed in claim 21,
**characterized in that** the readout is provided in the form of two arrows pointing in opposite directions.

23. The blood pressure measuring device as claimed in claim 21 or 22,
**characterized in that** the readout can be provided electronically only at predetermined instants of time, in particular prior to or during the blood pressure measurement.

24. The blood pressure measuring device as claimed in any one of the claims 12 to 23,
**characterized in that** provision is made for a measurement value storage and/or a device for determining the validity of the measurement results, hence enabling a readout of improper measurement conditions to be provided in response to the measurement position, the measurement inclination angle or any movement taking place during the measurement cycle.

25. The blood pressure measuring device as claimed in any one of the claims 12 to 24,
**characterized in that** the display device adapted to indicate the measurement result of the orientation sensing unit (5) is the same device that also enables indication of the blood pressure measurement values and a pulse rate.

## Revendications

1. Procédé pour déterminer la pression sanguine au niveau de l'avant-bras, selon lequel on place une unité d'application (1) comportant un capteur de pression et un dispositif d'affichage sur le poignet et on détecte l'orientation de l'avant-bras au moyen d'une unité de détection d'orientation (5), et selon lequel l'unité de détection d'orientation (5) délivre un signal électrique en fonction de l'orientation détectée de l'avant-bras et réalise le traitement ultérieur de ce signal électrique, et selon lequel une pression sanguine présente dans l'avant-bras est mesurée et l'orientation de l'avant-bras est détectée au moyen de l'unité de détection d'orientation (5) à l'intérieur d'un boîtier de l'appareil de mesure de la pression sanguine, et **caractérisé en ce que** le dispositif d'affichage de l'appareil de mesure de la pression sanguine est disposé sur le petit côté supérieur de l'unité d'application (1), pour le positionnement du dispositif d'affichage sur le petit côté supérieur du poignet, dans le prolongement du pouce.

2. Procédé selon la revendication 1, **caractérisé en ce que** la pression sanguine détectée est corrigée, dans l'unité d'évaluation (4), en fonction de l'orientation détectée de l'avant-bras.

3. Procédé selon la revendication 1, **caractérisé en ce que** la position angulaire (i) de l'avant-bras est détectée à l'aide d'un capteur d'inclinaison (5) et la pression sanguine détectée est corrigée en fonction de la position angulaire.

4. Procédé selon la revendication 1 ou 2, **caractérisé en ce qu'**un déplacement, notamment une vitesse ou accélération de l'avant-bras pendant la détection de la pression, est détecté et la pression sanguine détectée est corrigée en fonction du déplacement, notamment en fonction de la vitesse ou de l'accélération, de l'avant-bras.

5. Procédé selon au moins l'une des revendications précédentes, **caractérisé en ce que** le dispositif d'affichage délivre, d'une manière interactive avec l'utilisateur, comme indication une signalisation en retour indiquant si la position de mesure se situe dans une plage angulaire correcte de l'avant-bras, sur lequel s'effectue la mesure, et/ou indique de façon interactive de l'utilisateur à prendre la position de mesure correcte.

6. Procédé selon la revendication 5, **caractérisé en ce qu'**il est prévu comme indication des flèches dirigées vers le haut et vers le bas.

7. Procédé selon la revendication 6, **caractérisé en ce que** les flèches ou un clignotement indiquent précisément si l'utilisateur doit soulever ou abaisser de façon correspondante son poignet équipé de l'appareil de mesure la pression sanguine pour parvenir dans la position de mesure correcte.

8. Procédé selon l'une des revendications 5 à 7, **caractérisé en ce que** l'indication est affichée électroniquement uniquement à certains instants.

9. Procédé selon la revendication 8, **caractérisé en ce que** dans le cas d'une faible capacité indicatrice de la mesure de la pression sanguine en raison d'une position angulaire non appropriée de l'avant-bras, sur lequel est fixé l'appareil de mesure de la pression sanguine, la mesure de la pression sanguine est interrompue, l'interruption s'effectuent avant ou pendant la mesure de la pression sanguine.

10. Procédé selon l'une des revendications 1 à 9, **caractérisé en ce que** l'orientation de l'avant-bras s'effectue en parallèle, dans le temps, avec la détection de la pression sanguine.

11. Procédé selon l'une des revendications 1 à 10, **caractérisé en ce que** le dispositif d'affichage, sur lequel le résultat de mesure du dispositif de détection d'orientation (5) est indiqué, est le même que celui avec lequel les valeurs de mesure de la pression sanguine et un pouls sont affichés.

12. Appareil de mesure de la pression sanguine comportant un capteur de pression pour produire un signal de pression, une unité d'application destinée à appliquer le capteur de pression contre le poignet d'un avant-bras, et une unité d'évaluation (4) pour évaluer le signal de pression, dans lequel il est prévu une unité de détection d'orientation (5) pour détecter l'orientation de l'avant-bras, au moyen de laquelle un signal électrique peut être délivré en fonction de l'orientation de l'avant-bras, pour la poursuite du traitement, et dans lequel l'unité de détection d'orientation (5) est disposée à l'intérieur d'un boîtier de l'appareil de mesure de la pression sanguine, et **caractérisé en ce que** le dispositif d'affichage est disposé sur le côté de l'unité d'application (1) servant à positionner le dispositif d'affichage sur le côté étroit supérieur de l'avant-bras dans le prolongement du pouce.

13. Appareil de mesure de la pression sanguine selon la revendication 12, **caractérisé en ce que** l'unité d'évaluation (4) comporte une unité de correction servant à corriger le signal de pression conformément à l'orientation détectée.

14. Appareil de mesure de la pression sanguine selon la revendication 12, **caractérisé en ce que** l'unité de détection d'orientation comprend un capteur d'inclinaison (5), qui détecte l'inclinaison de l'avant-bras, sur lequel le capteur de pression (2) est placé.

15. Appareil de mesure de la pression sanguine selon la revendication 12, 13 ou 14, **caractérisé en ce qu'**une unité de détection de déplacement (5) est prévue pour détecter un déplacement, notamment une vitesse ou une accélération, de l'avant-bras, et l'unité d'évaluation (4) comporte une unité de correction servant a corriger le signal de pression conformément au déplacement détecté, notamment la vitesse ou l'accélération.

16. Appareil de mesure de la pression sanguine selon la revendication 15, **caractérisé en ce que** l'unité de détection de déplacement comprend le capteur d'inclinaison (5) et une unité de différenciation reliée à ce capteur.

17. Appareil de mesure de la pression sanguine selon au moins l'une des revendications 12 à 16, **caractérisé en ce que** l'unité de détection d'orientation (5) et le capteur de pression (2) sont reliées à l'unité d'évaluation (4) par l'intermédiaire d'une unité de commutation temporelle.

18. Appareil de mesure de la pression sanguine selon la revendication 17, **caractérisé en ce que** l'unité de commutation temporelle (6) est agencée sous la forme aiguillage de commutation ou d'un multiplexeur.

19. Appareil de mesure de la pression sanguine selon au moins l'une des revendications 12 à 16, **caractérisé en ce que** l'unité de détection de l'orientation (5) et le capteur de pression (2) sont reliés respectivement séparément à l'unité d'évaluation (4) de sorte que l'orientation du bras inférieur s'effectue en parallèle, dans le temps, à la détection de la pression sanguine.

20. Appareil de mesure de la pression sanguine selon l'une des revendications 12 à 19, **caractérisé en ce qu'**il est prévu une unité de mémoire (16) pour mémoriser des données de référence.

21. Appareil de mesure de la pression sanguine selon l'une des revendications 12 à 20, **caractérisé en ce qu'**il est prévu un dispositif d'affichage qui comporte des indications qui sont affichées de telle sorte qu'une plage angulaire correcte et/ou incorrecte ou un déplacement de l'appareil de mesure de la pression sanguine et/ou une indication concernant la position de mesure correcte sont délivrées.

22. Appareil de mesure de la pression sanguine selon la revendication 21, **caractérisé en ce qu'**il est prévu comme indications deux flèches dirigées dans des directions opposées.

23. Appareil de mesure de la pression sanguine selon la revendication 21 ou 22, **caractérisé en ce que** l'indication peut être affichée électroniquement uniquement à des instants déterminés, notamment avant ou pendant la mesure de la pression sanguine.

24. Appareil de mesure de la pression sanguine selon l'une des revendications 12 à 23, **caractérisé en ce qu'**il comporte une mémoire de valeurs de mesure et/ou un dispositif pour la détermination de validation des résultats de mesure, de sorte qu'une indication concernant les conditions de mesure défectueuses peut être délivrée pendant la mesure en fonction de la position de mesure, de l'angle d'inclinaison de mesure ou du déplacement.

25. Appareil de mesure de la pression sanguine selon l'une des revendications 12 à 24, **caractérisé en ce que** le dispositif d'affichage, sur lequel le résultat de mesure de l'unité de détection d'orientation (5) peut être affiché, est le même que celui avec lequel les valeurs de mesure de la pression sanguine et le pouls peuvent être affichés.
